Europäisches Patentamt

European Patent Office

Office européen des brevets

(11) Veröffentlichungsnummer: **0 022 236**
**B1**

(12) **EUROPÄISCHE PATENTSCHRIFT**

(45) Veröffentlichungstag der Patentschrift: **28.12.83**

(21) Anmeldenummer: **80103700.3**

(22) Anmeldetag: **30.06.80**

(51) Int. Cl.³: **C 07 C 59/125,**
C 07 C 69/003,
C 07 C 69/28,
C 07 C 67/26,
C 07 C 51/41, C 10 M 5/06,
C 10 M 5/14, C 10 M 7/10,
C 10 M 7/20, C 10 M 1/24

(54) Neue Lithiumseifen, ihre Verwendung als Dickungsmittel in Schmierölen bzw. Schmierfetten und sie enthaltende Schmierfette.

(30) Priorität: **09.07.79 DE 2927686**

(43) Veröffentlichungstag der Anmeldung:
**14.01.81 Patentblatt 81/2**

(45) Bekanntmachung des Hinweises auf die Patenterteilung:
**28.12.83 Patentblatt 83/52**

(84) Benannte Vertragsstaaten:
**DE FR GB IT**

(56) Entgegenhaltungen:
**DE - B - 1 122 654**
**DE - B - 1 271 882**
**US - A - 3 036 970**
**US - A - 3 985 662**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Schweizer, Dieter, Dr.**
**Achenbachstrasse 7**
**D-4000 Düsseldorf 1 (DE)**
Erfinder: **Johann, Glasl, Dr.**
**Baverter Strasse 58**
**D-5650 Solingen (DE)**

0 022 236

Neue Lithiumseifen, ihre Verwendung als Dickungsmittel in Schmierölen bzw. Schmierfetten und sie enthaltende Schmierfette

Die Lithiumseife der 12-Hydroxystearinsäure (Lithiumoxystearat) wird oft als Verdickungsmittel in Schmierfetten-eingesetzt. 12-Hydroxystearinsäure wird aus Ricinolsäure durch Hydrierung der Doppelbindung hergestellt. Dieser Schritt erfordert den Einsatz von Autoklaven oder sonstigen Druckapparaturen, die teuer und im Unterhalt aufwendig sind. Außerdem müssen alle Vorsichtsmaßnahmen für das Arbeiten mit Wasserstoff ergriffen werden. Ein weiterer Nachteil ist, daß Ricinolsäure oft starken Preisschwankungen unterworfen ist.

Aus der US—A—3 036 970 sind Schmiermittelzusammensetzungen bekannt, die Alkali- und Erdalkalimetallseifen von Akoxyhydroxyfettsäuren und Alkylmercaptohydroxyfettsäuren enthalten, wie sie aus epoxydierten Fettsäuren durch Umsetzung mit $C_1$—$C_3$-Alkanolen oder $C_1$—$C_3$-Alkylmercaptanen hergestellt werden können. Seifen von Acyloxyhydroxyfettsäuren und solche Seifen enthaltende Schmiermittel werden in dieser Druckschrift nicht erwähnt.

Die Erfindung hat sich die Aufgabe gestellt, eine Klasse von neuen substituierten hydroxylierten Lithiumseifen zur Verfügung zu stellen, die als Verdickungsmittel in Schmierfetten in vielgestaltiger Weise Verwendung finden können. Die neuen Lithiumseifen sollen erfindungsgemäß einerseits leicht zugänglich und dabei insbesondere auf Basis von Naturstoffen herstellbar sein, andererseits will die Erfindung die Möglichkeit schaffen, durch varierbare Substitution der neuen Lithiumseifen ihre Verdickungswirkung steuerbar zu machen. Die Erfindung will also durch Auswahl und Anpassung der jeweils bestimmten Konstitution einer neuen Lithiumseife im Sinne der Erfindung letztlich Einfluß auf bestimmte Eigenschaften des Schmierfetts nehmen und damit eine erhöhte Anpaßbarkeit der Schmierfetteigenschaften an den gewünschten Einsatzzweck ermöglichen.

Die Lösung dieser erfindungsgemäßen Aufgabenstellung geht von der überraschenden Feststellung aus, daß Lithiumseifen bestimmter vicinal substituierter Hydroxycarbonsäuren eine ausgeprägte Verdickungswirkung in Schmiermitteln bzw. Schmierfetten besitzen und daß dabei insbesondere die im Schmierfett letzlich auftretenden Stoffparameter durch die Variation der Substituenten an der Hydroxycarbonsäure stark beeinflußt wird.

Gegenstand der Erfindung sind dementsprechend in einer ersten Ausführungsform neue Lithiumseifen von innenständig ein- und/oder mehrfach hydroxylierten gesättigten und/oder ungesättigten Fettsäuren mit 16—22 C-Atomen, die in Nachbarstellung zu wenigstens einer Hydroxylgruppe mit einem geradkettigen oder verzweigten gegebenenfals ungesättigten Acyloxyrest mit bis zu 18 C-Atomen substituiert sind. Die Erfindung betrifft in einer weiteren Ausführungsform die Verwendung dieser neuen Lithiumseifen als Dickungsmittel in Schmierölen bzw. Schmierfetten. Schließlich sind Gegenstand der Erfindung Schmierfette auf Basis üblicher bei Normaltemperatur flüssiger Schmiermittelgrundlagen, die unter Mitverwendung der zuvor genannten neuen Lithiumseifen eingedickt worden sind.

Die innenständig ein- oder mehrfach hydroxylierten gesättigten und/oder ungesättigten Fettsäuren mit 16—22 C-Atomen, die in Nachbarstellung zu wenigstens einer Hydroxylgruppe mit einem geradkettigen oder verzweigten gegebenenfalls ungesättigten Acyloxyrest substituiert sind, sind auf einfache Weise zugänglich. Man erhält sie beispielsweise durch Epoxydation der Doppelbindung innenständig ungesättigter Carbonsäuren der angegebenen Kettenlänge mit anschließender Ringöffnung des Epoxids mit Carbonsäuren. Die letztlich zur Bildung der Lithiumseife einzusetzenden erfindungsgemäß vicinal substituierten Hydroxycarbonsäuren können aber auch auf anderen bekannten Wegen hergestellt worden sein, soweit sie nur die aufgeführten Strukturmerkmale enthalten.

In einer bevorzugten Ausführungform der Erfindung leiten sich die neuen Lithiumseifen von ein- und/oder mehrfach ungesättigten Monocarbonsäuren mit 16—22 C-Atomen ab, die aus Naturstoffen gewonnen worden sind. Talg und/oder pflanzliche Öle sind einer bevorzugte Quelle höher ungesättigter Carbonsäuren des angegebenen C-Zahl-Bereichs. Es können dabei vorausbestimmte definierte ungesättigte Carbonsäuren der genannten Art zum Einsatz kommen, gleichfalls ist es aber auch möglich, Säuregemische zu verwenden.

Die hier betroffenen Ausgangsmaterialien für die Herstellung der vicinal substituierten Hydroxycarbonsäuren besitzen gewöhnlich 1—3 Doppelbindungen, gegebenenfalls in Mischung in geringen Mengen zusätzlich vorliegende Fettsäuren höherer Ungesättigkeit stören jedoch nicht. Die als Ausgangsmaterial eingesetzten ungesättigten Carbonsäuren können unsubstituiert oder auch — wie beispielsweise im Fall der Ricinolsäure — mit einer Hydroxylgruppe substituiert sein. Besondere Bedeutung kann ungesättigten $C_{18}$-Säuren zukommen, wobei hier in erster Linie Ölsäure, Linolsäure, Linolensäure und die Ricinolsäure zu nennen sind. Daneben ist aber auch die Erucasäure als vergleichsweise einfach zugängliches Ausgangsmaterial zu nennen.

Die Epoxydation solcher ungesättigten Säuren ist bekannt und kann beispielsweise mit Percarbonsäuren oder $H_2O_2$/Metallsalzen erreicht werden. Bei geeigneter Reaktionsführung lassen sich die Epoxide leicht bei Temperaturen zwischen 20 und 100°C und Atmosphärendruck in hohen Ausbeuten herstellen. Die Lage der Doppelbindung ist ohne Einfluß. Beim Vorliegen mehrerer Doppelbindungen im Molekül können eine oder auch mehrere Doppelbindungen epoxydiert werden. Zum druckschriftlichen Stand der Technik wird bezüglich der Epoxydation beispielsweise verwiesen auf die

2

US—PS 2 485 160 sowie die Veröffentlichung Chemical Week, April 1963, Seiten 55—60 und 64.

Die Ringöffnung der so erhaltenen Epoxid-Carbonsäuren mit Säuren ist grundsätzlich bekannt, vergleiche hierzu beispielsweise Chemical Abstracts, Vol. 56, 2400i-2401a.

Die Ringöffnung mit Säuren verläuft in der Regel drucklos. Unter Mitverwendung geeigneter saurer oder basischer Katalysatoren sind in vielen Fällen Ausbeuten von 95 bis 100% erreichbar.

Zur Herstellung der erfindungsgemäß letztlich gewünschten Lithiumseifen vicinal substituierter Hydroxycarbonsäuren kann die Spaltung des bzw. der vorgebildeten Epoxydringe mit gesättigten oder ungesättigten geradkettigen oder verzweigten Monocarbonsäuren mit bis zu 18 C-Atomen erfolgen. Bevorzugt sind entsprechende Carbonsäuren mit wenigstens 2 C-Atomen, wobei es sich gezeigt hat, daß im Fall der hier entstehenden Acyloxy-Substitution in Nachbarstellung zur innenständigen Hydroxylgruppe die verdickende Wirkung der Lithiumseife mit zunehmendem Molekulargewicht ebenfalls zunimmt. Zur Herstellung von Schmierfetten zunehmend steiferen Charakters eignen sich also insbesondere Monocarbonsäuren höherer C-Zahlen innerhalb des angegebenen Bereichs, beispielsweise solche von $C_{10}$—$C_{18}$. Das folgende Beispiel verdeutlicht diese Tendenz:

Die Lithiumseife eines Umsetzungsproduktes von Epoxystearinsäure mit Caprylsäure ($C_8$) liefert ein Schmierfett auf mineralischer Grundlage (10% Verdickungsmittel) mit einer Ruhepenetration (DIN 51804) von 411. Das entsprechende Lithiumsalz aus der Umsetzung von Epoxystearinsäure mit Stearinsäure ($C_{18}$) liefert demgegenüber unter vergleichbaren Bedingungen einen Ruhepenetrationswert von 163.

In Anspassung an den jeweils gewünschten Verwendungszweck lassen sich damit erfindungsgemäß universell verwendbare Schmierfette herstellen, die z.B. durch einen weiten Bereich von Duktilität auch bei tiefer Temperatur und gleichzeitig hinreichend hohem Tropfpunkt gekennzeichnet sind.

Die erfindungsgemäß einstellbare Verdickungswirking wird — wie im Falle des Lithium-Oxystearats — durch die Lithiumseifen und nicht etwa durch die entsprechenden vicinal substituierten Hydroxycarbonsäuren geschaffen. Die Einarbeitung der Lithiumseife in das Schmierfett kann im Rahmen der Erfindung analog zur Herstellung von Schmierfetten auf Basis von 12-Hydroxystearinsäure erfolgen. So kann die mit Acyloxyresten substituierte Hydroxycarbonsäure bzw. ein entsprechendes Hydroxycarbonsäuregemisch als solches oder in Form eines reaktionsfähigen Derivates, insbesondere als Ester, zunächst in die zu verdickende Schmierfettbasis eingearbeitet und dann dort in situ in die Lithiumseife überführt werden. Zur Bildung der Lithiumseifen können zunächst mäßig erhöhte Temperaturen — beispielsweise bis 100°C — und in einer anschließenden Verfahrensstufe stärker erhöhte Temperaturen zum vollständigen Aufschmelzen der gebildeten Seife und ihrer homogenen Verteilung im Schmierfett eingesetzt werden. Es kann dabei mit einer absatzweisen Zugabe der flüssigen Schmierölbasis zum Reaktionsgemisch gearbeitet werden.

Für die Herstellung der Lithiumseifen von Hydroxycarbonsäuren mit vicinalen Acyloxysubstituenten kann der Einsatz der freien vicinal substituierten Hydroxycarbonsäure zur anschließenden Bildung der Lithiumseife besonders zweckmäßig sein, um eine unkontrollierte Esterspaltung an unerwünschter Stelle — nämlich am vicinalen Acyloxyrest — von vornherein auszuschließen. Hier wird dann in aller Regel die substituierte Hydroxycarbonsäure mit einer geeigneten Lithiumverbindung in situ im flüssigen Schmiermittel zur Umsetzung gebracht. Als besonders geeignete Lithiumverbindung wird Lithiumhydroxyd bzw. eine wässrige Lithiumhydroxydlösung eingesetzt.

Als reaktionsfähiges Derivat der vicinal substituierten Hydroxycarbonsäuren zur Verseifung mit Lithiumverbindungen eignen sich besonders die entsprechenden Ester. Ester der substituierten Carbonsäuren mit Alkoholen mit bis zu 8 Kohlenstoffatomen sind in der Regel bevorzugt. Wird die in situ Verseifung im Schmiermittel angestrebt, so kann die Verwendung von Estern niederer Alkohole, insbesondere $C_1$—$C_3$, besonders zweckmäßig sein. Bei der Verseifung des vicinal substituierten Hydroxycarbonsäureesters in situ im flüssigen Schmiermittel gelingt in diesen Fällen leicht die Entfernung des bei der Esterspaltung freiwerdenden Alkohols. Das Arbeiten mit Estern der substituierten Hydroxycarbonsäuren kann gegenüber der Verwendung der entsprechenden freien Säuren gewisse Vorteile mit sich bringen. So wird beispielsweise durch den Einsatz von Estern eine unerwünschte Kondensation der Hydroxycarbonsäure mit sich selbst zurückgedrängt.

In den erfindungsgemäßen Schmiermitteln können Lithiumseifen bestimmter einzelner vicinal substituierter Hydroxycarbonsäuren oder aber auch Mischungen mehrerer unterschiedlicher Lithiumseifen der genannten Art vorliegen. Dabei ist eine Variation in der Konstitution der in Mischung miteinander vorliegenden Seifen in mehrfacher Hinsicht möglich. Mischungen von Lithiumseifen sind beispielsweise darauf zurückzuführen, daß — wie zuvor angegeben — Gemische von verschiedenen ein- und/oder mehrfach ungesättigten Fettsäuren mit 16—22 C-Atomen zum Einsatz gekommen sind. Statt dieser Variation und/oder in Kombination mit ihr liegt aber auch die Mischung von Seifen mit unterschiedlichen Acyloxyresten im Rahmen des erfindungsgemäßen Handelns. Da jede bestimmte Lithiumseife eine charakteristische ihr eigene Kombination von Verdickungswirkungen zeigt, wird ersichtlich, welch breite Palette von Kombinationsmöglichkeiten im Rahmen des erfindungsgemäßen Handelns für die Herstellung von eingedickten Schmierfetten zur Verfügung gestellt wird.

Die Konzentration der Lithiumseife bzw. des Lithiumseifengemisches gemäß der Erfindung in Schmiermittel liegt üblicherweise im Bereich von 2 bis 25 Gew.-% bezogen auf das gesamte Schmiermittel, vorzugsweise im Bereich von 3 bis 10 Gew.-%. Die Konzentration der verdickenden

3

Komponenten kann von ihrer speziellen Verdickungswirkung abhängig sein. So können mit den stark verdickenden acyloxysubstituierten Hydroxycarbonsäuren mit höheren Acyloxyresten ($C_{14}$—$C_{18}$) vergleichsweise geringe Mengen Verwendung finden.

Auch beim Einsatz von Seifenmischungen kann von solchen Gesetzmäßigkeiten Gebrauch gemacht werden.

Als Flüssigphasen liegen in den erfindungsgemäß eingedickten Schmierfetten übliche Schmierölgrundlagen vor. Geeignet sind beispielsweise mineralische Schmieröle, etwa solche auf Erdölbasis und/oder synthetische Schmiermittel, beispielsweise auf Polyolesterbasis. Neben den erfindungsgemäß eingesetzten Verdickungsmitteln können die in der Schmierfetttechnik üblichen Zusätze, wie zusätzliche Gleitmittel, Komponenten zur Erhöhung des Lastaufnahmevermögens, Stabilisatoren, Reinigungsmittel und dergleichen Verwendung finden.

In den folgenden Beispielen wird die Herstellung von Schmierfetten mit einem ca. 10%igen Zusatz an Lithiumsalzen gemäß der Erfindung als Verdickungsmittel geschildert. Die Herstellung der Schmierfette erfolgt dabei durch Verseifung bzw. Salzbildung mit Lithiumhydroxyd, teils an den freien vicinal substituierten Hydroxycarbonsäuren, teils an den entsprechenden Methylestern. Als Vergleichsprodukte sind die Lithiumsalze der 12-Hydroxystearinsäure und der 9,10-Dihydroxystearinsäure in den anschließenden tabellarischsen Vergleich einbezogen.

Die Kenndaten der Schmierfette sind durch die Werte zur Ruhepenetration und Walkpenetration gemäß Din 51 804 sowie durch den Tropfpunkt gemäß Din 51 801 wiedergegeben. Im einzelnen gilt hier:

Ruhepenetration DIN 51 804

Die Probe wird mit dem Spatel unter möglichst geringer mechanischer Beanspruchung luftblasenfrei in das Prüfgefäß gefüllt. Die Spitze des Viertelkonus wird mit dem Penetrometer so eingestellt, daß sie die Oberfläche der im Prüfgefäß befindlichen Probe eben berührt. Für eine Prüfdauer von 5 Sekunden wird die Arretierung der Konusführungsstange gelöst, so daß der Viertelkonus unter dem Einfluß seines Gewichtes in die Probe einsinken kann. Die Einsinktiefe wird in Zehntel-Millimeter gemessen und anschließend auf die Konuspenetration umgerechnet.

Walkpenetration DIN 51 804

Die Schmierfette werden in einem Schmierfett-Kneter innerhalb einer Minute gleichmäßig 60 mal gewalkt, anschließend wird die Penetration wie oben beschrieben gemessen.

Bestimmung des Tropfpunktes DIN 51 801

Die in einem Nippel befindliche Probe wird erhitzt, bis sie genügend weich ist, so daß ein Tropfen aus dem Nippel abfällt.

Beispiele

Es wurden Schmierfette mit jeweils 10% Zusatz an Verdickungsmittel auf Basis von Lithiumsalzen der folgenden vicinal substituierten Hydroxycarbonsäuren hergestellt:

Beispiel 1: 9,10-Hydroxy-acetoxy-stearinsäure
Beispiel 2: Epoxystearinsäure, umgesetzt mit Caprylsäure
Beispiel 3: Epoxystearinsäure, umgesetzt mit Caprinsäure
Beispiel 4: Epoxystearinsäure, umgesetzt mit Laurinsäure
Beispiel 5: Epoxystearinsäure, umgesetzt mit Palmitinsäure
Beispiel 6: Epoxystearinsäure, umgesetzt mit Stearinsäure
Vergleichsbeispiel 1: 9,10-Dihydroxystearinsäure
Vergleichsbeispiel 2: 12-Hydroxystearinsäure

In der folgenden tabellarischen Zusammenstellung sind Angaben zur Beschaffenheit der jeweils eingesetzten Säure, zum Ansatz bei der Schmierfettherstellung (in allen Fällen wurde eine mineralische Schmierölgrundlage eingesetzt) und zum Aussehen sowie den Kenndaten der erhaltenen Schmierfette zusammengefaßt.

4

TABELLE

| | Beispiel 1 | Beispiel 2 | Beispiel 3 | Beispiel 4 | Beispiel 5 | Beispiel 6 |
|---|---|---|---|---|---|---|
| Aussehen der Säure | gelb-braune trübe, hochvisk. Flüssigkeit | dunkelbraunes, leicht trübes, flüssiges Prod. | dunkelbraunes, hochviskose Flüssigkeit | hellbraunes, klares flüssiges Produkt | braunes, festes Produkt | braunes, festes Produkt |
| Säurezahl | 98,7 | 125 | 118,9 | 112 | 106 | 98 |
| MG | 567 | 448 | 471 | 500 | 528 | 571 |
| Ansatzgrößen | | | | | | |
| Einsatzmenge an Säure | 50 g | 50 g | 50 g | 50 g | 50 g | 50 g |
| LiOH $H_2O$ | 3,7 g | 4,7 g | 4,5 g | 4,2 g | 4,0 g | 3,7 g |
| Mineralisches Schmieröl | 433 g | 433 g | 433 g | 433 g | 433 g | 433 g |
| Aussehen der Schmierfette | dickflüssiges, homogenes Schmierfett | gelbes, weiches, homogenes Schmierf. | sehr festes, homogenes Schmierf. | braunes, festes, homogenes Schmierf. | gelbes, festes, homogenes Schmierf. | dto. |
| Kenndaten der Schmierfette | | | | | | |
| Ruhepenetration | | 411 | 191 | 193 | 196 | 163 |
| Walkpenetration | | — | 249 | 221 | 203 | 198 |
| Tropfpunkt | | 163°C | 161°C | 173°C | 156°C | 156°C |

**0 022 236**

TABELLE (Fortsetzung)

|  | Vergleichs-beispiel 1 | Vergleichs-beispiel 2 |
|---|---|---|
| Aussehen der Säure | weiße, weiche Paste | weißes, festes geschupptes Produkt |
| SZ bzw. VZ | 166,1 | 196,0 |
| MG | 337 | 285 |
| Ansatzgrößen | | |
| Einsatzmenge an Säuren bzw. Ester: | 50 g | 50 g |
| Li OH.H$_2$O | 6,23 g | 7 g |
| Minerallisches Schmierol | 433 g | 433 g |
| Aussehen der Schmierfette | flüssiges, trübes braunes Schmierfett | festes, geschmei-diges Schmierfett |
| Kenndaten der Schmierfette: | | |
| Ruhepenetration | | 278 |
| Walkpenetration | | 276 |
| Tropfpunkt | | 197°C |

**Patentansprüche**

1. Neue Lithiumseifen von innenständig ein- und/oder mehrfach hydroxylierten gesättigten und/oder ungesättigten Fettsäuren mit 16—22 C-Atomen, die in Nachbarstellung zu wenigstens einer Hydroxylgruppe mit einem geradkettigen oder verzweigten, gegebenenfalls ungesättigten Acyloxyrest mit bis zu 18 C-Atomen substituiert sind.

2. Lithiumseifen nach Anspruch 1, dadurch gekennzeichnet, daß sie Reaktionsprodukte von epoxydierten ein- und/oder mehrfach ungesättigten Fettsäuren bzw. ihren an der Carboxylgruppe veresterten Derivaten mit geradkettigen oder verzweigten, gegebenenfalls ungesättigten Monocarbonsäuren mit bis zu 18 C-Atomen und anschließender Bildung der Lithiumseife sind.

3. Lithiumseifen nach Ansprüchen 1 und 2, dadurch gekennzeichnet, daß sie von natürlich vorkommenden ungesättigten Fettsäuren bzw. Fettsäuregemischen und dabei insbesondere von Ölsäure, Linolsäure, Linolensäure, Erascusäure und/oder Ricinolsäure bzw. ihren Estern abstammen.

4. Lithiumseifen nach Ansprüchen 1 bis 3, dadurch gekennzeichnet, daß sie durch Umsetzung der Epoxidgruppe (n) der epoxydierten Fettsäuren bzw. ihrer an der Carboxylgruppe veresterten Derivate mit gesättigten Carbonsäuren mit bis zu 18 C-Atomen hergestellt worden sind.

5. Verwendung der Lithiumseifen nach Ansprüchen 1 bis 4 als Dickungsmittel in Schmierölen bzw. Schmierfetten.

6. Ausführungsform nach Anspruch 5, dadurch gekennzeichnet, daß die Lithiumseifen in situ im Schmieröl bzw. Schmierfett aus den entsprechenden substituierten hydroxylierten Fettsäuren oder ihren an der Carboxylgruppe veresterten Derivaten hergestellt worden sind.

6

7. Schmierfette aus einer mit einem Verdickungsmittel eingedickten Flüssigphase mit Schmiermitteleigenschaften und gegebenenfalls enthaltend übliche Schmierfettzusätze, dadurch gekennzeichnet, daß sie als Verdickungsmittel wenigstens anteilweise eine oder mehrere der neuen Lithiumseifen nach Ansprüchen 1 bis 4 enthalten.

**Revendications**

1. Nouveaux savons de lithium d'acides gras saturés et/ou insaturés en $C_{16}$—$C_{22}$ mono- et/ou poly-hydroxylés en position interne, et substitués en position voisine d'au moins un groupe hydroxy par un radical acyloxy à chaîne droite ou ramifiée, éventuellement insaturé, contenant jusqu'à 18 atomes de carbone.

2. Savons de lithium selon la revendication 1, caractérisés en ce qu'ils consistent en produits de réaction d'acides gras mono- et/ou poly-insaturés époxydés ou de leurs dérivés estérifiés sur le groupe carboxyle avec des acides monocarboxyliques à chaîne droite ou ramifiée, éventuellement insaturés, contenant jusqu'à 18 atomes de carbone, et formation subséquente du savon de lithium.

3. Savons de lithium selon les revendications 1 et 2, caractérisés en ce qu'ils dérivent d'acides gras ou mélanges d'acides gras insaturés d'origine naturelle et en particulier de l'acide oléique, de l'acide linoléique, de l'acide linolénique, de l'acide érucique et/ou de l'acide ricinoléique ou leurs esters.

4. Savons de lithium selon les revendications 1 à 3, caractérisés en ce qu'ils ont été préparés par réaction du ou des groupes époxydes des acides époxydés ou de leurs dérivés estérifiés sur le groupe carboxyle avec des acides carboxyliques saturés contenant jusqu'à 18 atomes de carbone.

5. Utilisation des savons de lithium selon les revendications 1 à 4 en tant qu'agents épaississants dans des huiles lubrifiantes ou graisses lubrifiantes.

6. Mode de réalisation selon la revendication 5, caractérisé en ce que les savons de lithium ont été préparés in situ dans l'huile lubrifiante ou la graisse lubrifiante à partir des acides gras hydroxylés portant les substituants correspondants ou de leurs dérivés estérifiés sur le groupe carboxyle.

7. Graisses lubrifiantes consistant en une phase liquide à propriétés lubrifiantes épaissie par un agent épaississant et contenant le cas échéant des additifs usuels pour lubrifiants, caractérisées en ce qu'elles contiennent en tant qu'agents épaississants, en partie au moins, un ou plusieurs des nouveaux savons de lithium selon les revendications 1 à 4.

**Claims**

1. New lithium soaps of internally mono- and/or polyhydroxylated saturated and/or unsaturated fatty acids containing from 16 to 22 carbon atoms which, in the position adjacent at least one hydroxyl group, are substituted by a straight-chain or branched, optionally unsaturated acyloxy radical containing up to 18 carbon atoms.

2. Lithium soaps as claimed in Claim 1, characterized in that they are reaction products of epoxidized mono- and/or polyunsaturated fatty acids or derivatives thereof esterified on the carboxyl group with straight-chain or branched, optionally unsaturated monocarboxylic acids containing up to 18 carbon atoms and subsequent formation of the lithium soap.

3. Lithium soaps as claimed in Claims 1 and 2, characterized in that they emanate from naturally occurring unsaturated fatty acids or fatty acid mixtures and, more particularly, from oleic acid, linoleic acid, linolenic acid, erucic acid and/or ricinoleic acid or esters thereof.

4. Lithium soaps as claimed in Claims 1 to 3, characterized in that they are obtained by reacting the epoxide group(s) in the epoxidized fatty acids or their derivatives esterified on the carboxyl group with saturated carboxylic acids containing up to 18 carbon atoms.

5. The use of the lithium soaps claimed in Claims 1 to 4 as thickeners in lubricating oils and lubricating greases.

6. The use claimed in Claim 5, characterized in that the lithium soaps are produced in situ in the lubricating oil or lubricating grease from the corresponding substituted hydroxylated fatty acids or their derivatives esterified on the carboxyl group.

7. A lubricating grease of a liquid phase with lubricant properties thickened with a thickener and optionally containing standard additives for lubricating greases, characterized in that they contain, at least partly, one or more of the new lithium soaps claimed in Claims 1 to 4 as thickener.